# EUROPÄISCHE PATENTANMELDUNG

(11) **EP 1 460 055 A1**
(43) Veröffentlichungstag der Anmeldung: **22.09.2004**
(21) Anmeldenummer: 04004639.3
(22) Anmeldetag: 01.03.2004
(51) Int. Cl.: C07C 68/00, C07C 69/96

(54) **Verfahren zur Herstellung eines Diarylcarbonats, wobei das Katalysatorsystem termisch in einem separaten Reaktionsapparat aktiviert wird**

(30) Priorität: 07.03.2003 DE 10309954
(71) Anmelder: Bayer MaterialScience AG, 51368 Leverkusen (DE)
(72) Erfinder: Dahlmann, Marc, Dr., 40219 Düsseldorf (DE); Fischer, Peter, Dr., 50676 Köln (DE); Hansen, Sven-Michael, Dr., 51373 Leverkusen (DE); Reisinger, Claus-Peter, Dr., Wixom MI 48393 (US)

(57) **Zusammenfassung**

Die vorliegende Erfindung betrifft ein Verfahren zur Herstellung eines Diarylcarbonats durch Direktcarbonylierung von Hydroxyaromaten in Gegenwart eines Katalysatorsystems, wobei das Katalysatorsystem durch geeignete thermische Vorbehandlung in einem separaten Reaktionsapparat aktiviert wird.

## Beschreibung

Die vorliegende Erfindung betrifft ein Verfahren zur Herstellung eines Diarylcarbonats (DAC) durch Direktcarbonylierung von Hydroxyaromaten in Gegenwart eines Katalysatorsystems, wobei das Katalysatorsystem durch geeignete thermische Vorbehandlung in einem separaten Reaktionsapparat aktiviert wird.

Die Herstellung von DAC durch oxidative Direktcarbonylierung von aromatischen Hydroxyverbindungen in Gegenwart von Kohlenmonoxid (CO), Sauerstoff (O₂) und in Gegenwart eines Edelmetall-Katalysators ist bekannt. Als Edelmetall wird bevorzugt Palladium eingesetzt. Zusätzlich können ein Cokatalysator (z. B. Mangan-, Kupfer, Blei-, Titan- oder Kobaltsalze), eine Base, Bromidquellen, quaternäre Salze, verschiedene Chinone bzw. Hydrochinone und Trockenmittel eingesetzt werden. Dabei kann in einem Lösungsmittel gearbeitet werden.

US-A 2002/0177724 offenbart ein mehrstufiges Verfahren zur Herstellung von Diarylcarbonaten aus aromatischen Hydroxyverbindungen durch deren Umsetzung mit Kohlenmonoxid und Sauerstoff, wobei in einer ersten Stufe in Gegenwart von Kohlenmonoxid, jedoch in Abwesenheit von Sauerstoff gearbeitet wird.

EP-A 0 654 461 offenbart, dass die Carbonylierung reproduzierbar und mit höheren Raum-Zeit-Ausbeuten durchgeführt werden kann, wenn das Reaktionsgemisch mit Kohlenmonoxid _{"}aktiviert", d.h. vor der Reaktionsdurchführung für einen gewissen Zeitraum mit reinem Kohlenmonoxid begast wird.

Das Verfahren lieferte jedoch keine für eine technische Umsetzung zufriedenstellenden Ausbeuten und erzeugte größere Mengen Nebenprodukte. Es ist daher die Aufgabe der vorliegenden Erfindung, ein Verfahren bereitzustellen, das eine hohe Ausbeute und eine hohe Produktqualität, das heißt Reinheit des Produktes, liefert.

Es wurde nun ein Verfahren gefunden, welches eine überraschend hohe Selektivität und Ausbeute ermöglicht. Das heißt, dass die Menge der Nebenprodukte niedrig ist und dass die Ausbeute an Produkt hoch ist. Wesentlich an diesem erfindungsgemäßen Verfahren ist, dass das verwendete Katalysatorsystem durch thermische Vorbehandlung (nachfolgend auch als thermische Aktivierung oder Tempern bezeichnet) in einem separaten Reaktionsapparat aktiviert wird.

Gegenstand der vorliegenden Erfmdung und Lösung der genannten Aufgabe ist also ein Verfahren gemäß Anspruch 1.

In einer besonderen Ausführungsform der vorliegenden Erfindung umfasst die genannte zweite Vorrichtung, welche nachfolgend auch als Aktivierungsapparat bezeichnet wird, mehrere einzelne Vorrichtungen.

Weitere besondere Ausführungsformen des erfindungsgemäßen Verfahrens sind in den Unteransprüchen wiedergegeben.

Bei den erfindungsgemäß umsetzbaren aromatischen Hydroxyverbindungen der Formel (II)

R-O-H (II)

handelt es sich beispielsweise um Monohydroxyverbindungen wie Phenol, o-, m- oder p-Kresol, o-, m- oder p-Chlorphenol, o-, m- oder p-Ethylphenol, o-, m- oder p-Propylphenol, o-, m- oder p-Methoxyphenol, 2,6-Dimethylphenol, 2,4-Dimethylphenol, 3,4-Dimethylphenol, 1-Naphthol, 2-Naphthol oder Di- bzw. Polyhydroxyverbindungen wie Resorcin und Hydrochinon, sowie Tris- und Bisphenole wie 2,2-Bis-(4-hydroxyphenyl)-propan (Bisphenol A), 2,2- Bis-(3,5-dimethyl-4-hydroxyphenyl)-propan, 1,1-Bis-(4-hydroxyphenyl)-cyclohexan, 1,1-Bis-(4-hydroxyphenyl)-3,3,5-trimethylcyclohexan oder 6,6'-Dihydroxy-3,3,3',3'-tetramethyl-1,1'-spiro(bis)-indan, 2,4'-Hydroxybiphenyl oder 4,4'-Hydroxybiphenyl. Allgemein handelt es sich im Falle einer Substitution der aromatischen Hydroxyverbindung um 1 bis 3 Substituenten in der Bedeutung von C₁-C₁₈-Alkyl, C₆-C₁₈-Aryl, C₇-C₁₈-Aralkyl, C₁-C₁₈-Alkoxy, Fluor, Chlor oder Brom. Bevorzugt werden Monohydroxyverbindungen eingesetzt, besonders bevorzugt ist Phenol.

Die für das erfmdungsgemäße Verfahren geeignete Katalysatorkomponente d) bestehen bevorzugt aus mindestens einem Edelmetall der Gruppe VIII, vorzugsweise Palladium. Es kann bei dem erfmdungsgemäßen Verfahren in verschiedener Form zugegeben werden. Palladium kann in metallischer Form z.B. als Palladiumschwarz oder auf einem Träger wie Pd/C, Pd/Al₂O₃, Pd/SiO₂ oder bevorzugt in Form von Palladium-Verbindungen der Oxidationsstufen 0 und +2, wie beispielsweise Palladium(11)-acetylacetonat -halogenide, -carboxylate von C₂-C₁₈-Carbonsäuren, -dicarboxylate wie Oxalat, -nitrat, -sulfat, -oxide oder Palladiumkomplexe, die beispielsweise Kohlenmonoxid, Olefine, Amine, Nitrile, Phosphorverbindungen und Halogenide enthalten können, eingesetzt werden. Besonders bevorzugt sind Palladiumbromid und Palladiumacetylacetonat.

Die Menge an Katalysatorkomponente d) ist im erfmdungsgemäßen Verfahren nicht beschränkt. Bevorzugt wird so viel Katalysator zugesetzt, dass die Konzentration des Metalls im Reaktionsansatz 1 bis 3000 ppm beträgt, besonders bevorzugt sind Konzentrationen von 5 bis 500 ppm.

Als Katalysatorkomponente e), die als Cokatalysator wirkt, wird ein Metall der Gruppen III A, III B, IV A, IV B, V B, I B, II B, VI B, VII B, der Seltenerdmetalle (Atomnummern 58-71) oder der Eisengruppe des Periodensystems der Elemente (Mendelejew), gegebenenfalls auch Mischungen davon, verwendet, wobei das Metall in verschiedenen Oxidationsstufen eingesetzt werden kann. Die Dokumente US-A 5,142,086, US-A 5,231,210, US-A 5,284,964, EP-A 0 350 697, EP-A 0 350 700 und US-A 5,336,803 offenbaren den Einsatz derartiger Elemente als Cokatalysatoren.

Bevorzugt werden Pb, Ti, Mn, Cu, Co, V, Zn, Ce und Mo eingesetzt. Ohne das erfmdungsgemäße Verfahren einzuschränken, seien Blei (II), Mangan(II), Mangan(III), Kupfer(I), Kupfer(II), Kobalt(II), Kobalt(III), Vanadium(III) und Vanadium(IV) genannt. Die Metalle können beispielsweise als Halogenide, Oxide, Carboxylate von C₂-C₁₈-Carbonsäuren, Diketonate oder Nitrate sowie als Komplexverbindungen eingesetzt werden, die beispielsweise Kohlenmonoxid, Olefine, aromatische und aliphatische Monoamine oder Polyamine, Phosphorverbindungen, Pyridine, Bipyridine, Terpyridine, Chinoline, Isochinoline, Kryptanden, Schiffbasen und Halogenide enthalten können. Besonders bevorzugt werden Mn, Cu, Mo, Ti, Pb und Ce eingesetzt. Ganz besonders bevorzugt werden Manganverbindungen im erfmdungsgemäßen Verfahren verwendet, besonders bevorzugt Mangan(II)- und Mangan(III)komplexe, ganz besonders bevorzugt Mangan(II)acetylacetonat bzw. Mangan(III)-acetylacetonat, sowie Mangan(II)bromid.

Die Katalysatorkomponente e) (Cokatalysator), die auch in situ gebildet werden kann, wird bevorzugt in einer solchen Menge zugesetzt, dass seine Konzentration im Bereich von 0,0001 bis 20 Gew.-% des Reaktionsgemisches liegt. Bevorzugt ist der Konzentrationsbereich von 0,001 bis 5 Gew.-%, besonders bevorzugt 0,005 bis 2 Gew.-%.

Als optionale Komponenten f) des Katalysatorsystems können beispielsweise Bromidverbindungen (V-a), Basen (V-b) oder Lösungsmittel (V-c) eingesetzt werden.

Bei den im Rahmen der vorliegenden Erfindung eingesetzten Bromidverbindungen (V-a) handelt es sich beispielsweise um die Alkalibromide oder Erdalkalibromide, bevorzugt aber um die Bromidsalze von organischen Kationen. Bei den organischen Kationen kann es sich beispielsweise um mit organischen Resten substituierte Ammonium-, Guanidinium-, Phosphonium- oder Sulfoniumsalze, gegebenenfalls auch Mischungen davon, handeln. Geeignet für den Einsatz in das erfindungsgemäße Verfahren sind Ammonium-, Guanidinium-, Phosphonium- und Sulfoniumsalze, die als organische Reste C₆- bis C₁₀-Aryl-, C₇- bis C₁₂-Aralkyl- und/oder C₁- bis C₂₀-Alkyl-Reste enthalten. Bevorzugt werden in das erfmdungsgemäße Verfahren Ammoniumsalze eingesetzt, die als organische Reste C₆- bis C₁₀-Aryl-, C₇- bis C₁₂-Aralkyl- und/oder C₁- bis C₂₀-Alkyl-Reste tragen, besonders bevorzugt sind Tetrabutylammoniumbromid und Tetrabutylphosphoniumbromid. Die Menge eines solchen quaternären Salzes kann beispielsweise 0,1 bis 20 Gew.-%, bezogen auf das Gewicht des Reaktionsgemisches, betragen. Vorzugsweise beträgt diese Menge 0,5 bis 15 Gew.-%, besonders bevorzugt 1 bis 5 Gew.-%.

Für das erfmdungsgemäße Verfahren einsetzbare Basen (V-b) sind beispielsweise Alkalihydroxide, Alkalisalze bzw. quaternäre Salze von schwachen Säuren wie Alkali-tert.-butylate oder Alkalisalze bzw. quaternäre Salze von aromatischen Hydroxyverbindungen der Formel (II), in der R die oben angegebene Bedeutung hat. Ganz besonders bevorzugt wird ein Alkalisalz bzw. quaternäres Salz der aromatischen Hydroxyverbindung der Formel (II) verwendet, die auch zum aromatischen Carbonat der Formel (I) umgesetzt werden soll, beispielsweise Tetrabutylammonium- oder Kaliumphenolat.

Die Alkalisalze können Lithium-, Natrium-, Kalium-, Rubidium- oder Cäsiumsalze sein. Bevorzugt werden Lithium-, Natrium- und Kaliumphenolate, besonders bevorzugt Kaliumphenolat, eingesetzt.

Die quaternären Salze können Ammonium-, Phosphonium-, Pyridinium-, Sulfonium- oder Guanidiniumsalze sein, die als organische Reste C6- bis C₁₈-Aryl-, C₇- bis C₁₈-Aralkyl- und/oder C₁- bis C₂₀-Alkyl-Reste besitzen. Die Reste können alle gleich oder verschieden sein, gegebenenfalls können auch Mischungen mehrerer Quartärsalze eingesetzt werden. Bevorzugt wird dabei gegebenenfalls das selbe Kation eingesetzt, das auch als Bromid für Komponente (V-a) verwendet wird. Weiterhin bevorzugt werden Tetraphenylphosphonium, Tetrabutylammonium-, Tetrabutylphosphonium, besonders bevorzugt wird Tetrabutylammonium.

Alternativ können auch Trialkylaminbasen wie Tributylamin, Diisopropylethylamin, 1,5-Diazabicyclo[4.3.0]non-5-en (DBN) oder 1,8-Diazabicyclo[5.4.0]undec-7-en (DBU) verwendet werden.

Die Base (V-b) wird in einer von der Stöchiometrie unabhängigen Menge zugesetzt. Das Verhältnis von Platinmetall, z.B. Palladium, zu Base wird vorzugsweise so gewählt, dass pro mol Platinmetall, z.B. Palladium, 0,1 bis 5000, bevorzugt 1 bis 1000, besonders bevorzugt 10 bis 300 Äquivalente Base eingesetzt werden.

Optional können unter den Reaktionsbedingungen inerte Lösungsmittel (V-c) verwendet werden. Als Beispiele für Lösungsmittel seien aliphatische Kohlenwasserstoffe wie Pentan, Petrolether, Cyclohexan, Isooctan, aromatischen Kohlenwasserstoffe wie Benzol, Toluol, Xylole, Chloraromaten wie Chlorbenzol oder Dichlorbenzol, Ether, wie Dioxan, Tetrahydrofuran, t-Butyl-methylether, Anisol, Amide wie Dimethylacetamid, N-Methylpyrrolidinon, Alkohole wie t-Butanol, Cumylalkohol, Isoamylalkohol, Diethylenglykol, Tetramethylharnstoff genannt. Es können Gemische von Lösungsmitteln eingesetzt werden. Das inerte Lösemittel kann mit einem Anteil von 1 bis 99 %, bevorzugt 20 bis 98 %, besonders bevorzugt 30 bis 98 %, in der Reaktionsmischung enthalten sein. Insbesondere bei der Verwendung von anorganischen Komponenten V-a oder V-b ist der Einsatz von Lösungsmitteln, welche die Löslichkeit von anorganischen Salzen wie NaBr oder NaOPh vermitteln wie z.B. dipolar aprotische Lösungsmittel (z.B. N.N-Dimethylformamid, N,N-Dimethylacetamid, N-Methylpyrrolidinon, Sulfolan, Acetonitril) bzw. Kronenether, Cryptanden oder _{"}offene Kronenether" (z. B. Diethylenglykoldimethylether, Triethylenglykoldimethylether, Tetraethylenglykoldimethylether) günstig.

Die thermische Aktivierung des Katalysatorsystems nach dem erfindungsgemäßen Verfahren erfolgt durch thermische Vorbehandlung von Mischungen, die unabhängig voneinander Anteile von etwa 1 bis 100 Gew.-% der Gesamtmenge der jeweiligen Komponenten a), d) und e) enthalten. Der Anteil der Komponente d) sollte bevorzugt möglichst groß gewählt werden, bevorzugt größer als etwa 50 Gew.-% der Gesamtmenge, besonders bevorzugt größer als etwa 80 Gew.-%, ganz besonders bevorzugt etwa 90 bis 100 Gew.-% der Gesamtmenge der Komponente d). Der Anteil der Komponente e) sollte bevorzugt möglichst groß gewählt werden, bevorzugt größer als etwa 50 Gew.-% der Gesamtmenge, besonders bevorzugt größer als etwa 80 Gew.-%, ganz besonders bevorzugt etwa 90 bis 100 Gew.-% der Gesamtmenge der Komponente e). Die Anteile der Einzelkomponenten können auch noch während der Aktivierung durch Zudosierung von mindestens einer Komponente geändert werden. Zusätzlich kann die Komponente f) mit einem Anteil von etwa 1 bis 100 Gew.-% der Gesamtmenge bei der thermischen Aktivierung enthalten sein. Der Anteil der Komponente (V-a) sollte bevorzugt etwa 1 bis 100 Gew.-% der Gesamtmenge, besonders bevorzugt etwa 5 bis 100 Gew.-% der Gesamtmenge.

Die Masse der erfindungsgemäß thermisch aktivierten Komponenten kann also im Mittel etwa 1 bis 100 % der Gesamtmasse des Reaktionsgemisches betragen. Um die Dimensionierung des Reaktionsapparates möglichst klein zu halten, werden bevorzugt nur Teilmassen von kleiner als 75 % der Gesamtmasse, besonders bevorzugt kleiner 40 % der Gesamtmasse in den oder die Aktivierungsapparat(e), d.h. in die zweite Vorrichtung, dosiert und die Restmenge erst in den oder die darauffolgenden Reaktionsapparat(e) eingeleitet.

CO und O₂ und optionale weitere Trägergase können im Rahmen der Beschränkungen durch die Explosionsgrenzen bei der thermischen Aktivierung in beliebigem Verhältnis zueinander eingesetzt werden. Bevorzugt ist das CO/O₂-Verhältnis bei der thermischen Aktivierung in etwa gleich oder größer als das in der Reaktion eingesetzte Verhältnis.

Der Druck beträgt bevorzugt etwa 0,01 bis 500 bar. Bevorzugt wird ein Druck verwendet, der kleiner oder gleich dem mittleren Reaktionsdruck ist.

Der Zeitraum t2, in dem die erfmdungsgemäße thermische Aktivierung durchgerührt wird, beträgt normalerweise etwa 1 Minute bis 10 Stunden und umfasst einen Anteil von weniger als 50 %, bevorzugt weniger als 35 % der sich anschließenden Gesamtreaktionszeit t1 oder mittleren Verweilzeit.

Die thermische Aktivierung wird bei Temperaturen T2 durchgeführt, die im Mittel etwa mehr als 15°C, bevorzugt mehr als 25°C, besonders bevorzugt mehr als 35°C unter der mittleren Reaktionstemperatur T1 liegen.

Unter der mittleren Aktivierungstemperatur T2 wird im Sinne der Erfmdung der Quotient aus dem Integral der Auftragung der Aktivierungstemperatur gegen die Aktivierungszeitdauer (bzw. Verweilzeit) und der Aktivierungszeitdauer (bzw. Verweilzeit) verstanden.

Unter der mittleren Reaktionstemperatur T1 wird im Sinne der Erfindung der Quotient aus dem Integral der Auftragung der Reaktionstemperatur gegen die Reaktionszeitdauer (bzw. Verweilzeit) und der Reaktionszeitdauer (bzw. Verweilzeit) verstanden.

Die mittlere Aktivierungstemperatur T2 liegt normalerweise etwa im Bereich von 10 bis 120°C.

Verschiedene Temperaturprofile sind möglich. Die Aktivierung kann bei einer konstanten Temperatur, bei einer Reihe verschiedener Temperaturen, beispielsweise einer in äquidistanten oder verschieden langen Schritten steigenden Treppenfunktion oder verschiedenen anderen linearen, konvexen oder konkaven Temperatur-Zeit-Profilen durchgeführt werden. Verschiedene solcher Zeit-Temperaturprofile können zu einem Gesamtprofil kombiniert werden. Bevorzugt werden konstante Temperaturen oder Temperatur-Zeit-Profile mit monoton positiver Steigung verwendet. Besonders bevorzugt weicht dabei die Endtemperatur bei der Temperung weniger als 5°C von der Reaktionsanfangstemperatur ab.

Da sich an die Aktivierungszeitdauer im Normalfall direkt die Reaktion anschließt, ist typischerweise die Endtemperatur der Aktivierung auch die Anfangsreaktionstemperatur, welche normalerweise durch Temperaturregelung oder durch Einstellen stationärer Zustände konstant gehalten wird. Eine Aktivierung bei einer konstanten Temperatur wird im Sinn der Erfmdung deshalb als eine Kombination aus einem Temperatur-Zeit-Profil mit der Steigung 0 und einer Hochheizphase, d.h. ein im Rahmen der Güte der Regelung monotones Ansteigen von dieser gewählten konstanten Temperatur zur Anfangsreaktionstemperatur verstanden.

Das erfindungsgemäße Verfahren kann sowohl in kontinuierlicher wie auch diskontinuierlicher Weise geführt werden. Bei einer diskontinuierlichen Durchführung kann die Aktivierung in einem separaten Aktivierungsapparat (z.B. einem Behälter mit Gaseinleitungsrührer), von dem aus die Aktivierungsmischung vor oder - in Portionen oder kontinuierlich - während der Reaktion dosiert wird, erfolgen. Bei einer kontinuierlichen Durchführung wird das für die Aktivierung bestimmte Gemisch bevorzugt durch einen geeigneten Apparat (beispielsweise einen gerührten Behälter, eine Blasensäule oder eine Kombination aus einer oder mehreren Düsen und einer Rohrstrecke) geleitet, der so dimensioniert ist, dass die mittlere Verweilzeit weniger als 50 % der Verweilzeit der eigentlichen Reaktionsapparate beträgt.

Das erfindungsgemäße Verfahren zur Bildung des aromatischen Carbonats (Formel I) wird bevorzugt bei einer mittleren Reaktionstemperatur von 30 bis 200°C, bevorzugt 50 bis 150°C, besonders bevorzugt 60 bis 130°C, und bevorzugt bei einem mittleren Reaktionsdruck von 1 bis 200 bar, bevorzugt 1 bis 50 bar, besonders bevorzugt 1 bis 10 bar durchgeführt. Bevorzugt wird bei einem konstanten Druck oder einem Zeit-Druck-Stufenprofil mit verschiedenen konstanten Drücken oder verschiedenen konstanten Druckgradienten gearbeitet. Bevorzugt wird bei einer konstanten Temperatur oder einem Zeit-Temperatur-Stufenprofil mit verschiedenen konstanten Temperaturen oder verschiedenen konstanten Temperaturgradienten gearbeitet.

Die Zusammensetzung der Reaktionsgase Kohlenmonoxid und Sauerstoff kann in der Reaktionsphase in weiten Konzentrationsgrenzen variiert werden, es wird jedoch zweckmäßigerweise ein CO:O₂-Molverhältnis (normiert auf CO) von 1:0,001 bis 1:1, bevorzugt 1:0,01 bis 1:0,5 und besonders bevorzugt von 1:0,02 bis 1:0,3 eingestellt. Der Sauerstoffpartialdruck ist bei diesen Molverhältnissen groß genug, um hohe Raum-Zeit-Ausbeuten erreichen zu können.

Alle Ausgangsverbindungen können mit Verunreinigungen aus ihrer Herstellung und Lagerung kontaminiert sein, jedoch ist es im Sinne der Reinheit des Endproduktes wünschenswert, mit möglichst sauberen Chemikalien zu arbeiten. Auch die Reaktionsgase unterliegen keinen besonderen Reinheitsanforderungen. So kann Synthesegas als CO-Quelle und Luft als O₂-Träger dienen, es ist jedoch darauf zu achten, dass keine Katalysatorgifte wie z.B. Schwefel oder dessen Verbindungen eingetragen werden. Die Gase können mit einem oder mehreren anderen Gasen wie Stickstoff, Argon, Kohlendioxid oder Wasserstoff verdünnt sein. In der bevorzugten Ausführungsform des erfmdungsgemäßen Verfahrens werden reines CO und reiner Sauerstoff verwendet.

Das Katalysatorsystem kann entweder homogenen oder heterogen aufgebaut sein. Heterogene Katalysatorsysteme, bei denen die Katalysatorkomponente d, z.B. das Platinmetall, und/oder der Cokatalysator (die Katalysatorkomponente e) auf einem heterogenen Träger aufgebracht sind, können z.B. als Pulver oder Formkörper eingesetzt werden. Die übrigen optionalen Komponenten des Katalysatorsystems (die Komponenten f), wie die Base, die quaternäre Verbindung, sowie gegebenenfalls der Cokatalysator, sind weiterhin in der Reaktionslösung homogen gelöst. Die Menge des Platinmetalls am Gesamtgewicht des heterogenen Katalysators beträgt 0,01 bis 15 Gew.-%, bevorzugt 0,05 bis 10 Gew.-%, gerechnet als Platinmetall.

Als Cokatalysatoren auf dem Katalysatorträger wird mindestens eine Metallverbindung der oben genannten Art eingesetzt.

Die Menge des Cokatalysators am Gesamtgewicht des heterogenen Katalysators beträgt bevorzugt 0,01 bis 15 Gew.-%, bevorzugt 0,05 bis 10 Gew.-% gerechnet als Metall.

Als Katalysatorträger eignen sich ein oder mehrere Metalloxide aus der Gruppe von V, Mn, Ti, Cu, Zr, La, der Seltenerdmetalle (Atomnummern 58-71), sowohl im Sinne chemisch einheitlicher Reinsubstanzen als auch im Gemisch, sowie Eisen- und Kobaltoxide, Nickel-, Aluminium-, Silizium- und Magnesiumoxid, Zeolithe und Aktivkohlen. Wird der Trägerkatalysator als Pulver eingesetzt, sind zur Vermischung der Reaktionskomponenten die zu verwendenden Rührbehälter mit dafür brauchbaren Rührern ausgestattet, bzw. als Blasensäulenreaktor gestaltet.

Beim Arbeiten mit Trägerkatalysator-Pulvern als Suspension in Rührgefäßen oder Blasensäulen werden bevorzugt Mengen von 0,001 bis 50 Gew.-%, bevorzugt von 0,01 bis 20 Gew.-%, besonders bevorzugt von 0,1 bis 10 Gew.-% Trägerkatalysator-Pulver auf die eingesetzte Menge an aromatischer Hydroxyverbindung, verwendet.

In bevorzugten Ausführungsformen wird der heterogene Trägerkatalysator ortsfest in Rührbehältern, einer Blasensäule, einem Rieselphasenreaktor oder Kaskaden dieser Reaktoren eingesetzt. Eine Abtrennung des Trägerkatalysators entfällt dann völlig.

Als Reaktoren für das erfmdungsgemäße Verfahren mit homogenem oder heterogenem Katalysator sind Rührkessel, Autoklaven und Blasensäulen geeignet, wobei diese als Einzelreaktoren oder als Kaskade eingesetzt werden können. In einer Kaskade können 2 bis 15, bevorzugt 2 bis 10, besonders bevorzugt 2 bis 5 Reaktoren hintereinandergeschaltet sein.

Zur Vermischung der Reaktionskomponenten sind die erfindungsgemäß bevorzugt zu verwenden Rührbehälter mit dafür geeigneten Rührer ausgestattet. Solche Rührer sind dem Fachmann bekannt. Es seien beispielhaft genannt: Scheiben-, Impeller-, Propeller-, Schaufel-, MIG- und Intermig-Rührer, Rohrrührer und verschiedene Hohlrührertypen. Bevorzugte Rührer sind solche, die eine effektive Vermischung von Gasen und Flüssigkeiten erlauben, beispielsweise Hohlrohrbegasungsrührer, Propellerrührer etc.

Als Blasensäulen können in dem erfmdungsgemäßen Verfahren folgende Typen, eingesetzt werden: einfache Blasensäulen, Blasensäulen mit Einbauten, wie z.B.: Blasensäulen mit parallelen Kammern, Kaskaden-Blasensäulen mit Siebböden oder Einlochböden, Blasensäulen mit Packungen, mit statischen Mischern, pulsierende Siebbodenblasensäulen, Schlaufenreaktoren wie z.B.: Mammutschlaufenreaktoren, Abstromschlaufenreaktoren, Strahlschlaufenreaktor, Freistrahlreaktoren, Strahldüsenreaktoren, Blasensäulen mit Flüssigkeitstauchstrahler, Abstrom-Aufstrom-Blasensäulen und weitere dem Fachmann bekannte Blasensäulenreaktoren. Solche Apparat sind beispielsweise offenbart in Chem. Ing. Tech. 51 (1979) Nr. 3, S. 208-216 und in W-D. Deckwer, Reaktionstechnik in Blasensäulen, Otto Salle Verlag 1985.

In einer bevorzugten Ausführungsform kommen Blasensäulenreaktoren und Blasensäulen-Kaskaden zum Einsatz, die eine effektive Vermischung von Gas und Flüssigkeiten erlauben, wie beispielsweise Kaskaden-Blasensäulen und Schlaufenreaktoren. Zur Aufrechterhaltung einer guten Durchmischung von Flüssigkeit und Reaktionsgas können Verteilungs- und Redispergierorgane entlang der Längsachse der Blasensäulenreaktoren angebracht sein. Als feste Redispergierorgane kommen Einlochböden, Lochplatten, Siebböden, sowie weitere dem Fachmann bekannte Einbauten zum Einsatz. Für die Erstdispergierung des Reaktionsgases in der flüssigen Phase bei der Eindosierung sind übliche Vorrichtungen wie poröse Sinterplatten, Lochplatten, Siebböden, Einsteckrohre, Düsen, Begasungsringe und weitere dem Fachmann bekannte Dispergiervorrichtungen einsetzbar.

Das erfmdungsgemäße Verfahren kann in verschiedenen Ausführungsvarianten ausgeübt werden. Eine Möglichkeit besteht in im folgenden beschriebenen besonderen Form der diskontinuierlichen Durchführung. Dabei werden CO und Sauerstoff entweder durch einen Begasungsrührer, wie im Falle eines Rührkessels, oder durch andere bekannte Gasverteilungsorgane in das zu aktivierende Gemisch, bestehend aus den Komponenten a), d) und e) sowie ggf. f), geleitet. Nach Erreichen des optimalen Umsatzes wird das Reaktionsgemisch aus dem Reaktor entfernt oder gegebenenfalls im Reaktor aufgearbeitet, z.B. filtriert. Im Falle der Verwendung pulverförmiger Trägerkatalysatoren können diese aus der Reaktionsmischung z.B. durch Filtration, Sedimentation oder Zentrifugieren abgetrennt werden.

In diskontinuierlichen Versuchen verwendete Trägerkatalysatoren können bei gleichen Einsatzstoffen gegebenenfalls ohne Reinigung wiederholt eingesetzt werden. Bei kontinuierlicher Arbeitsweise können die eingesetzten Trägerkatalysatoren über lange Zeit im Reaktor verbleiben und gegebenenfalls regeneriert werden.

In bevorzugter Weise kommt eine kontinuierliche Arbeitsweise im Einzelreaktor oder in einer Kaskade mehrerer Reaktoren zum Einsatz. Bei Verwendung ortsfester heterogener Katalysatoren können diese über lange Zeit im Reaktor verbleiben und dort auch gegebenenfalls regeneriert werden.

### Beispiele

Die Reaktionskomponenten wurden durch Gaschromatographie (GC) untersucht, wobei die Massen der Komponenten mittels eines internen Standards bestimmt werden konnten. Aus den im Abstand mehrerer Stunden erhaltenen Daten wurden Mittelwerte und Standardabweichungen bestimmt. Die Selektivität wurde berechnet, indem die Menge des in der Reaktionsmischung enthaltenen Restphenols und des zu Diphenylcarbonat (DPC) umgesetzten Phenols addiert und durch die eingesetzte Gesamtphenolmenge dividiert wurden. Das dabei nicht erfasste Phenol wurde zu Nebenprodukten umgesetzt.

### Beispiel 1 Bsp. 1)

Aus drei verschiedenen Vorlagen (1. Vorlage: Palladiumbromid, Tetrabutylammoniumbromid, Chlorbenzol, 2. Vorlage: Tetrabutylamnoniuimbromid, Tetrabutylammoniumphenolat, Phenol, Chlorbenzol, 3. Vorlage: Mangantrisacetylacetonat, Chlorbenzol) wurde ein Reaktionsstrom der Zusammensetzung 0,05 g/h Palladiumbromid, 0,2 g/h Mangantrisacetylacetonat, 10 g/h Tetrabutylammoniumbromid, 9 g/h Tetrabutylammoniumphenolat, 79,95 g/h Phenol, 8,8 g/h GC-Standard und 241 g/h Chlorbenzol in einen Katalysatoraktivierbehälter (RA1) mit einer Temperatur von 50°C und einer mittleren Verweilzeit von 43 Minuten dosiert. Bei einem Druck von 3 bar wurde er mit 100 N1/h (100 Norm-Liter pro Stunde) einer Gasmischung aus Kohlenmonoxid und Sauerstoff (99:1 Vol.-%) begast. In den Austrag wurde aus der dritten Vorlage nochmals eine Teilmenge nachdosiert, so dass ein Gesamtstrom der Zusammensetzung 0,05 g/h Palladiumbromid, 0,8 g/h Mangantrisacetylacetonat, 10 g/h Tetrabutylammoniumbromid, 9g/h Tetrabutylammoniumphenolat, 79,95 g/h Phenol, und 391,3 g/h Chlorbenzol resultierte. Dieser Strom wurde in einen gerührten Reaktionsbehälter (RA2) eingeleitet, der bei einem Druck von 3 bar mit 300 Nl/h einer Gasmischung aus Kohlenmonoxid und Sauerstoff (97:3 Vol.-%), begast wurde. Er wurde mit einer Reaktionstemperatur von 100°C und einer mittleren Verweilzeit von 127 Minuten verfahren. Im Austrag des Reaktionsapparates (RA2) und im Austrag des Katalysatoraktivierbehälters (RA1) wurden nach Einstellung des stationären Zustands GC-Proben genommen. Die Ergebnisse sind in Tabelle 1 dargestellt.

### Vergleichsbeispiel 1 (Vgl. 1)

Aus drei verschiedenen Vorlagen (1. Vorlage: Palladiumbromid, Tetrabutylammoniumbromid, Chlorbenzol, 2. Vorlage: Tetrabutylamnoniuimbromid, Tetrabutylammoniumphenolat, Phenol, Chlorbenzol, 3. Vorlage: Mangantrisacetylacetonat, Chlorbenzol) wurde ein Reaktionsstrom der Zusammensetzung 0,05 g/h Palladiumbromid, 0,8 g/h Mangantrisacetylacetonat, 10 g/h Tetrabutylammoniumbromid, 9g/h Tetrabutylammoniumphenolat, 80,03 g/h Phenol, und 391,2 g/h Chlorbenzol in einen gerührten Reaktionsbehälter (RA2) eingeleitet, der bei einem Druck von 3 bar mit 300 Nl/h einer Gasmischung aus Kohlenmonoxid und Sauerstoff (97 : 3 Vol.-%), begast wurde. Er wurde mit einer Reaktionstemperatur von 100°C und einer mittleren Verweilzeit von 129 Minuten verfahren. Im Austrag des Reaktionsapparates (RA2) wurden nach Einstellung des stationären Zustands GC-Proben genommen. Die über die anschließende Laufzeit gemittelten Ergebnisse sind in Tabelle 1 dargestellt.

**Tabelle 1:**

| Vergleich von Selektivitäten und DPC-Ausbeuten | | | |
|---|---|---|---|
| | Probennahmestelle Reaktor | Selektivität Phenol [%] | DPC [Gew.%] |
| Bsp.1 | RA1 | | 0,33±0,02 |
| | RA 2 | 94,0±0,2 | 7,59±0,30 |
| Vergl. 1 | RA 2 | 87,9±2,4 | 5,95±0,25 |

Es zeigt sich, dass, obwohl in dem separaten Katalysatoraktivierungsbehälter (RA1) kaum Umsatz stattfindet, sowohl die Selektivität als auch die Katalysatoraktivität bei selber Verweilzeit im Austrags des Reaktors (RA2) höher sind.

## Patentansprüche

1. Verfahren zur Herstellung eines aromatischen Carbonats der Formel (I)
R-O-CO-O-R (I),
wobei R für einen aromatischen Rest steht,
umfassend die Umsetzung
a) einer aromatischen Hydroxyverbindung der Formel (II)
R-O-H (II)
mit
b) Kohlenmonoxid und
c) Sauerstoff
in Gegenwart eines Katalysatorsystems enthaltend
d) eine oder mehrere verschiedene Verbindungen ausgewählt aus der Gruppe bestehend aus den Verbindungen von Ru, Os, Rh, Ir, Ni, Pd und von Pt (Katalysatorkomponente d) genannt) und
e) eine oder mehrere verschiedene Verbindungen ausgewählt aus der Gruppe bestehend aus den Verbindungen von Sc, Y, La, Ti, Zr, Hf, V, Nb, Ta, Cr, Mo, W, Mn, Fe, Ni, Co, Cu, Ag, Au, Zn, Cd, Hg, Ga, In, Tl, Ge, Sn, Sb, Bi und von Pb (Katalysatorkomponente e) genannt),
wobei die Umsetzung für eine Zeit t1 und bei einer mittleren Reaktionstemperatur T1 in einer ersten Vorrichtung (die Reaktionsapparat genannt wird) durchgeführt wird,
und wobei zumindest ein Teil der insgesamt zur Umsetzung kommenden aromatischen Hydroxyverbindung der Formel (II) und zumindest ein Teil der insgesamt bei der Umsetzung gegenwärtigen Katalysatorkomponente d und zumindest ein Teil der insgesamt bei der Umsetzung gegenwärtigen Katalysatorkomponente e zusammen vor der Umsetzung für eine Zeit t2 von weniger als 50 % der Zeit t1 bei einer Temperatur T2, die im Mittel mindestens 15°C unter der mittleren Reaktionstemperatur T1 liegt in Gegenwart von Kohlenmonoxid und Sauerstoff in einer zweiten Vorrichtung getempert werden.

2. Das Verfahren nach Anspruch 1, wobei 50 bis 100 Gew.-% der insgesamt bei der Umsetzung gegenwärtigen Katalysatorkomponente d) und 10 bis 100 Gew.-% der insgesamt bei der Umsetzung gegenwärtigen Katalysatorkomponente e zusammen vor der Umsetzung in Gegenwart von Kohlenmonoxid und Sauerstoff in einer zweiten Vorrichtung getempert werden.

3. Das Verfahren nach Anspruch 1 oder 2, wobei die Umsetzung in Gegenwart eines Lösungsmittels erfolgt.

4. Das Verfahren nach einem der Ansprüche 1 bis 3, wobei das Katalysatorsystem zusätzlich
f) weitere Komponenten ausgewählt aus der Gruppe bestehend aus Bromidverbindungen, Basen und Lösungsmittel
enthält.

5. Das Verfahren nach einem der Ansprüche 1 bis 4, wobei t2 kleiner ist als 35 % von t1.

6. Das Verfahren nach einem der Ansprüche 1 bis 5, wobei T2 im Mittel mindestens 25°C unter T1 liegt.

7. Das Verfahren nach einem der Ansprüche 1 bis 6, wobei die Temperung bei einer konstanten Temperatur T2 durchgeführt wird.

8. Das Verfahren nach einem der Ansprüche 1 bis 6, wobei die Temperung mit einem Temperatur-Zeit-Profil mit monoton positiver Steigung durchgeführt wird.

9. Das Verfahren nach einem der Ansprüche 1 bis 8, wobei die Endtemperatur bei der Temperung weniger als 5°C von der Reaktionsanfangstemperatur abweicht.

10. Das Verfahren nach einem der Ansprüche 1 bis 9, wobei das aromatische Carbonat der Formel (I) Diphenylcarbonat ist.

11. Das Verfahren nach einem der Ansprüche 1 bis 10, wobei das Verfahren kontinuierlich durchgeführt wird.

12. Das Verfahren nach Anspruch 11, wobei der Massestrom, der die zweite Vorrichtung durchströmt kleiner ist als 75 % des Massestromes, der die erste Vorrichtung durchströmt.
